# EUROPEAN PATENT SPECIFICATION

(11) **EP 1 767 169 B1**
(45) Date of publication and mention of the grant of the patent: **04.03.2009**
(21) Application number: 06077122.7
(22) Date of filing: 01.09.2000
(51) Int. Cl.: A61F 2/06

(54) **Tubular stent-graft composite device and method of manufacture**
Röhrenförmige Stent-Graft-Kompositvorrichtung und Verfahren zu ihrer Herstellung
Dispositif composite de greffe d'endoprothèse tubulaire et son procédé de fabrication

(30) Priority: 01.09.1999 US 151833 P
(43) Date of publication of application: 28.03.2007
(62) Divisional of application: 00959672.7
(73) Proprietor: Boston Scientific Scimed, Inc., Maple Grove, MN 55311 (US)
(72) Inventor: Tseng, David, Cupertino, CA 95014 (US); Parson, Bruce A., Pompano Beach, FL 33060-9133 (US); Kiraly, William E., Carlsbad, CA 92009-7035 (US); Golds, Ellen, Hastings-on-Hudson, NY 10706 (US); Hill, Jason, Brooklyn Park, MN 55443 (US)
(74) Representative: Hauck Patent- und Rechtsanwälte

(56) References cited:
- EP-A1- 0 605 243
- WO-A-97/25938
- WO-A-98/38947
- WO-A2-98/26731
- US-A- 5 749 880

## Description

### FIELD OF THE INVENTION:

The present invention relates generally to a tubular implantable prosthesis including a stent and graft composite structure used to repair and/or replace a body vessel. More particularly, the present invention relates to a multi-layered stent-graft composite device including a radially expandable stent and a graft formed of both a sheet of PTFE and an extruded PTFE tube.

### BACKGROUND OF THE INVENTION:

It is well known to employ various endoprostheses for the treatment of diseases of various body vessels. Such endoprostheses are used to repair, replace or otherwise hold open a blocked or occluded body lumen such as that found in the vascular system.

One type of endoprosthesis is commonly referred to as a stent. A stent is a generally longitudinal tubular device formed of biocompatible material which is useful to open and support various lumens in the body. For example, stents may be used in the vascular system, urogenital tract and bile duct, as well as in a variety of other applications in the body. Endovascular stents have become widely used for the treatment of stenosis, strictures or aneurysms in various blood vessels. These devices are implanted within the vessel to open and/or reinforce collapsing or partially occluded sections of the vessel. Often, stents may be used in conjunction with a graft with provides additional support for blood flow through weakened sections of the blood vessel.

Various stent constructions are well known for such purposes. A common feature of such stent construction is that the stent includes an elongate tubular configuration having open spaces therethrough which permit the radial expansion of the stent. Stents generally are open ended and are radially expandable between a generally unexpanded insertion diameter and an expanded implantation diameter which is greater than the unexpanded insertion diameter. Stents are often flexible in configuration, which allows them to be inserted through and conform to tortuous pathways in the blood vessels. The stent is generally inserted in a radially compressed state and expanded either through a self-expanding mechanism, or through the use of balloon catheters. For example, various stent constructions and their method of deployment are shown in U.S. Patent Nos. 4,503,569 to Dotter; 4,733,665 to Palmaz; 4,856,561 to Hillstead; 4,580,568 to Gianturco; 4,732,152 to Wallsten and 4,886,062 to Wiktor.

Another implantable prosthesis which is commonly used in the vascular system is a vascular graft. Grafts are typically used to repair or replace damaged portions of the blood vessel. Grafts are elongate tubular members exhibiting sufficient blood tightness to permit the graft to serve as a replacement for the damaged vessel. If the graft is thin enough and has adequate flexibility, it may be collapsed and inserted into a body vessel at a location within the body having diameter smaller than that of the intended repair site. An intraluminal delivery device, such as a catheter, is then used to move the graft into the repair site and expand the diameter of the graft therein to conform with the diameter of the vessel. In this manner, the graft provides a new blood contacting surface within the vessel lumen. The grafts are also microporous so as to permit tissue ingrowth and cell endotheliazation therealong. This improves the patency of the graft and promotes long term healing.

Vascular grafts may be formed of various materials such as synthetic textile materials. Grafts may also be formed of fluoropolymers such as expanded polytetrafluoroethylene (ePTFE). Grafts formed of ePTFE have the requisite degree of blood tightness yet exhibit a microstructure defined by interconnected nodes and fibrils which promotes tissue ingrowth and cell endotheliazation.

PTFE tubular structures for use as grafts may be formed in one or two manners. Tubes of PTFE may be formed in an extrusion process. The extruded tubes of PTFE may then be expanded and sintered to form expanded PTFE (ePTFE) exhibiting the requisite node and fibril structure. Extruded tubes formed of ePTFE have well documented characteristics of blood tightness and porosity and also provide significant radial strength. Such radial strength enables the extruded PTFE tube to be used in combination with a radially expandable stent so as to form a stent/graft composite device. However, extrusion is impractical for the manufacture of thin walled tubes. As wall thickness decreases, processing by free extrusion becomes more difficult because the resulting tube is prone to kink, collapse or flatten during handling. Also, with free extrusion it is more difficult to control the inner diameter of the tube within tight tolerances which is required for medical applications.

Tubular PTFE structures may also be formed of sheets of ePTFE. Such sheets may be subsequently formed into a tubular configuration and applied to a stent to function in a stent/graft environment. Use of PTFE sheets is beneficial in that sheets can be formed to have thinner wall thicknesses than conventional extruded tubes. Such thinner wall thicknesses enable the stent/graft composite device to be more easily intraluminally implanted by use of a delivery device.

This feature is especially important in the formation of a stent/graft composite device where a stent is provided with a PTFE cover about the exterior of the stent and a liner disposed about the interior surface of the stent. Thus, these composite devices have the beneficial aspects of a stent which is used to hold open a blocked or occluded vessel, and also a graft which is used to replace or repair a damaged vessel. While such composite device employing tubular structures formed of PTFE sheets are particularly beneficial due to the thinness at which they may be formed, these sheets may suffer from the lack of radial strength provided by extruded tubes. Thus, it may be difficult to maintain an internal liner and an external cover both formed from sheets where the stent must undergo contraction and expansion which is necessary to deliver and deploy the composite device at its ultimate location in the blood vessel.

Examples of the use of ePTFE endoprostheses are shown in U.S. Patent Nos. 5,700,285, 5,735,892 and 5,810,870, all of which are issued to Myers et al.. Each of the Myers patents discloses a stent-graft composite device wherein a tubular diametrically adjustable stent having has an exterior surface, a luminal surface, and either or both of an exterior and luminal tubular covering of porous ePTFE. Each of the tubular coverings exhibits a longitudinal seam therealong which extends from an exterior surface to a luminal surface thereof. Individual thin films of ePTFE are used to form the tubular coverings such that the combined thickness thereof, exclusive of the stent, is less than about 0.10 mm. The Myers devices rely upon the inherent thinness of the films to provide a compact structure for intraluminal implantation.

An additional example is shown in commonly assigned U.S. Patent No. 5,800,512 to Lentz et al.. The Lentz patent discloses an implantable microporous ePTFE tubular vascular graft which includes a first ePTFE tube and a second ePTFE tube circumferentially disposed thereover. The first ePTFE tube exhibits a porosity sufficient to promote cell endotheliazation therealong. The second ePTFE tube exhibits enhanced radial tensile strength in excess of that of the first tube. Together, the tubes provide a graft device having an overall improved radial tensile strength and exhibiting increased porosity.

The procedures which utilize the above disclosed devices obviate the need for major surgical intervention and reduce the risks associated with such a procedure. It is desirable, however, to provide a stent-graft composite device which exhibits sufficient radial strength to permit the composite device to accommodate a radially expandable stent and yet be of sufficient thinness so as to permit intraluminal delivery by a conventional intraluminal delivery device.
The exhibition of sufficient radial strength enables the stent to withstand contraction and expansion within a biological lumen, while sufficient thinness enables insertion of the stent at a remote surgical site and subsequent movement into a small diameter body vessel.

WO98/38947 discloses a stent-graft composite intraluminal prosthetic device according to the preamble of claim 1 and a method according to the preamble of claim 8. This known device comprises:
an elongated radially adjustable tubular stent, defining opposed interior and exterior stent surfaces;
a PTFE stent cover positioned about the exterior surface of said stent; and
a PTFE stent liner positioned about the interior surface of said stent;
wherein one of said cover and said liner is formed of a seamless extruded tube and the other of said cover and said liner is formed of a sheet.

### SUMMARY OF THE INVENTION

It is therefore an object of the present invention to provide an improved tubular-stent graft composite device.

It is another object of the present invention to provide a stent-graft composite device having increased radial strength to accommodate a radially expandable stent.

It is a further object of the present invention to provide a stent-graft composite device having sufficient radial thinness so as to permit intraluminal delivery by a delivery device, such as a catheter.

In the efficient attainment of these and other objectives, the present invention provides a composite stent-graft tubular prosthesis according to claim 1. A radially expandable stent is interposed between the inner and outer PTFE tubular structures. The interposed stent is formed from an elongated wire with a plurality of longitudinal spaces in an open tubular configuration. The outer tubular structure comprises an extruded PTFE tube wherein the inner tubular structure comprises an elongated PTFE sheet having longitudinal edges which are joined together to form a tubular structure.

A method of making a stent-graft luminal prosthesis of the present invention is also disclosed. The method provides for the formation of a first PTFE tubular structure wherein the first tubular structure is a PTFE sheet having a tubular configuration. A stent is positioned over said first PTFE tubular structure, the stent being a tubular configuration formed of an elongated wire. A second PTFE structure is then formed over said stent, with the second PTFE structure being an extruded PTFE tube.

### BRIEF DESCRIPTION OF THE DRAWINGS

Figure 1 is a perspective view of a preferred embodiment of a tubular stent-graft composite device of the present invention.
Figure 2 is a perspective view of an elongated extruded PTFE tube of the type used in the composite device of Figure 1.
Figure 3(a) is a cross-section of an elongated extruded PTFE tube of Figure 2 along section a-a.
Figure 3(b) is a transverse cross-section of an elongated extruded PTFE tube of Figure 2 taken along the line b-b.
Figure 4 is a perspective view of a sheet of PTFE of the type used in the composite device of Figure 1.
Figure 5 is a perspective view of a sheet of PTFE of Figure 4 formed into a tubular configuration (not according to the invention).
Figure 6 is a side view of one embodiment of a stent which may be used in a stent-graft composite structure of the present invention.
Figure 7 is an exploded perspective view showing one embodiment of the stent-graft composite device of the present invention.
Figure 8 is a transverse cross-section of the composite device shown in Figure 7.

### DETAILED DESCRIPTION OF THE PREFERRED EMBODIMENTS

The following is a description of the preferred embodiments of the present invention, in which like elements are identically numbered.

The prosthesis of the present invention is a multi-layered composite tubular structure which is particularly suited for use as an endoprosthesis or vascular graft. In particular, an outer tubular cover is disposed about an inner tubular liner with a stent disposed therebetween. The stent is a tubular structure formed of an elongated wire having a plurality of open longitudinal spaces. Each of the tubular cover and liner is formed of polytetrafluoroethylene (PTFE), as PTFE exhibits superior biocompatibility. Further, PTFE is particularly suitable for vascular applications as it exhibits low thrombogenicity. Tubes formed of extruded PTFE may be expanded to form expanded polytetrafluoroethylene (ePTFE) tubes, where the ePTFE tubes have a fibrous state which is defined by elongated fibrils interconnected by spaced apart nodes. Also, ePTFE may be formed into elongated sheets having longitudinal edges which are joined together to form a tubular structure. In the present invention, the inner liner is formed of such an ePTFE sheet and the outer cover comprises an ePTFE tube. The liner and cover may be adheringly secured to one another with a thermoplastic adhesive or laminated together through the open spaces of the stent.

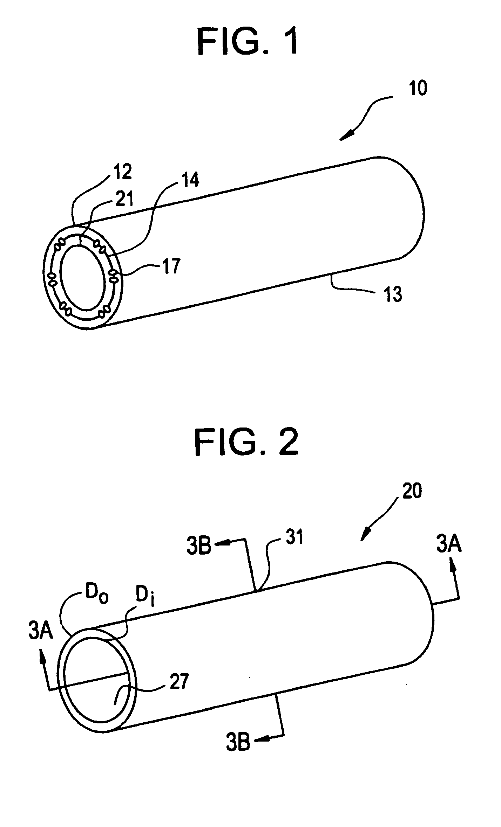

An improved tubular stent-graft composite device 10 of the present invention is shown in Figure 1. Prosthesis 10 is a tubular structure having an elongated body 13. Prosthesis 10 comprises an outer tubular cover 12, an inner tubular liner 14 and a tubular stent 17 positioned therebetween. Stent 17 shown herein is just one example of the plurality of stent configurations which may be employed within the present invention.

A stent-graft composite device of the present invention is constructed by initially forming a first inner tubular liner 14, which is a PTFE sheet. Stent 17 is then positioned over the inner tubular liner 14. An outer PTFE tubular cover 12 is then formed over stent 17, outer tubular cover being formed of an extruded PTFE tube. PTFE tubular structures, whether extruded tubes or wrapped PTFE sheets, show advantageous biophysical compatibility qualities.

In a preferred embodiment of the present invention as described hereinbelow, tubular cover 12 comprises an extruded PTFE tube and tubular liner 14 comprises an ePTFE sheet having a pair of longitudinal edges which are joined together to form at seam 21 a tubular configuration. Once extruded, a tube of the type shown in cover 12 is expanded to form an ePTFE tube. The tube is expanded using differing process parameters (i.e., rates, deformation levels, temperature, etc.) to develop a desired microporous structure. The specifically designed structure of the resulting composite tube has defined properties of strength and porosity which yield a prosthesis 10 having long term patency and good healing characteristics, as well as superior radial strength characteristics.

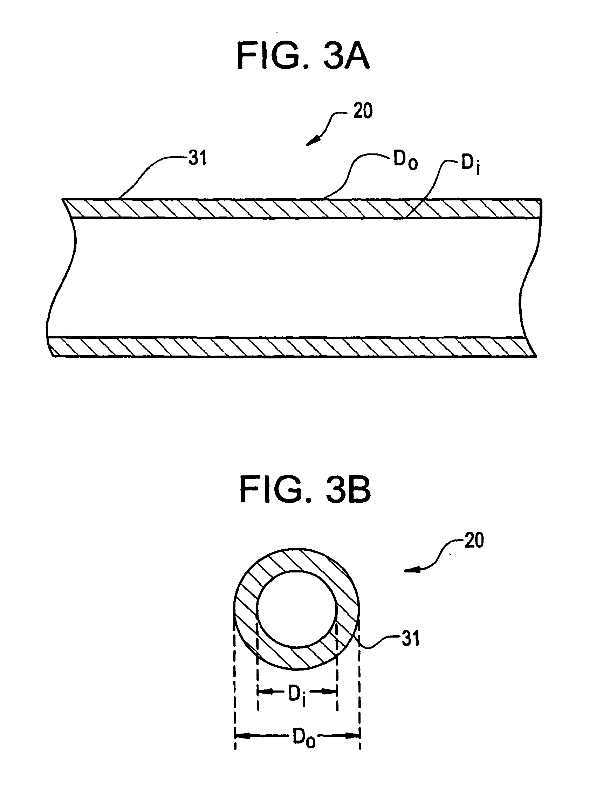

Now the individual elements of an improved tubular stent-graft device of a preferred embodiment of the present invention may be described. Referring now to Figures 2, 3(a) and 3(b), an ePTFE tube 20 has an elongated body 31 and a longitudinal axis defined therethrough. Elongated body 31 is generally cylindrical and is defined by a tubular configuration wherein an inner diameter Dᵢ and an outer diameter Dₒ define the thickness of the body. As shown in Figure 3(a), tube 20 has a uniform thickness extending along the length of body 31. As depicted in Figure 3(b), a transverse cross section of tube 20 at any point along the length thereof will show a nonvarying outer diameter Dₒ and a non-varying inner diameter Dᵢ.

Tube 20 may be formed in a conventional extrusion process wherein a preform of PTFE and a lubricant are extruded through a tubular extruder barrel creating a tubular structure. The lubricant is removed from the extrudate, which is then expanded by stretching in a direction parallel to the longitudinal axis of the tubular structure at a temperature less than the melt point of PTFE. Typically, the tubular structure is longitudinally expanded by an expansion ratio of more than 2:1 (i.e. at least two times its original length). After completion of longitudinal expansion, the tubular structure is then sintered to effect amorphous-locking of the PTFE polymer.

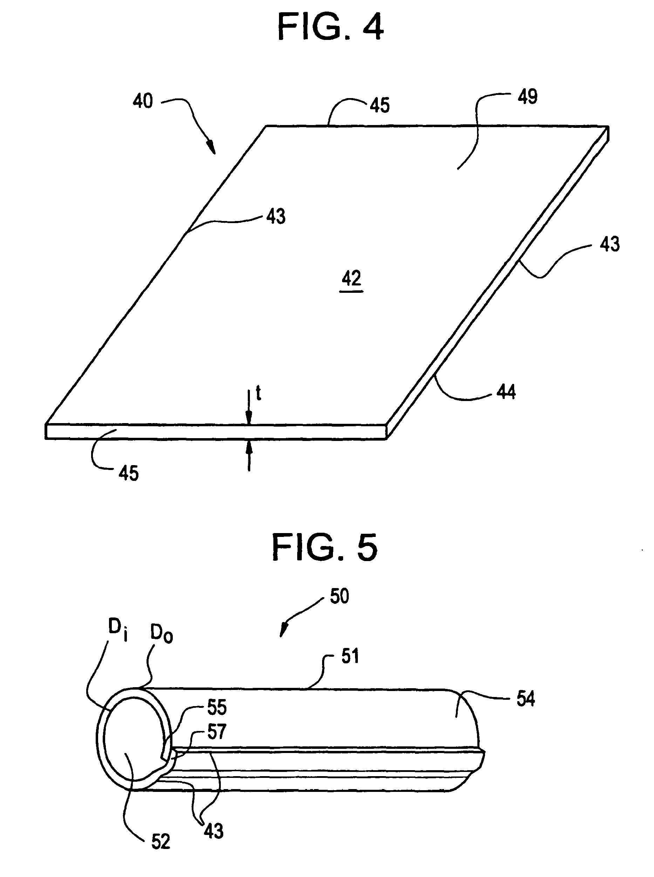

Referring now to Figures 4 and 5, an ePTFE sheet or film 40 is shown. Sheet 40 includes a pair of longitudinal edges 43 and opposing edges 45 which define the parameters of a substantially planar body 49 therebetween. Body 49 has an upper surface 42 and an opposed lower surface 44, each of which is generally smooth. Body 49 is further defined by a thickness t which is sufficient for delivery of a prosthesis 10 (shown in Figure 1) into a vessel via an intraluminal delivery device.

Sheet 40 is then rolled into a tubular structure 50, shown in Figure 5. The tubular structure 50 may be formed by wrapping sheet 40 around a mandrel and forming a seam by overlapping the longitudinal edges of the sheet not according to the invention. After the seam is formed, the mandrel is placed into an oven which is set at a temperature above the melt-point of the sheet. The mandrel remains in the oven until the edges sufficiently adhere to one another. After heating, the mandrel is removed from the oven and allowed to cool. The mandrel is then removed from within the resulting tubular graft. Longitudinal edges 43 of sheet 40 can alternatively be joined together at seam 55 by a thermoplastic adhesive such as fluorinated ethylene propylene (FEP), creating a short overlap 57 (not according to the invention).

Tubular structure 50 has an outer surface 54 which corresponds to lower surface 44 of sheet 40 and a luminal surface 52 which corresponds to upper surface 42. Tubular structure 50 has a generally elongated body 51 with opposed open ends. Outer diameter Dₒ and inner diameter Dᵢ of tube 50 defined by the thickness of sheet 40 wherein Dₒ -Dᵢ = t.

In the case of the present invention where the tubular structure 50 makes up the inner layer of prosthesis 10, longitudinal edges 43 need not be joined together so as to overlap. In such an embodiment (not shown), an adhesive is placed on the contacting surfaces of longitudinal edges 43 and melted just enough to bond edges 43 to one another.

The method of manufacturing the present stent-graft device comprises many steps performed by various types of machinery. First, a green tube of PTFE is formed through a conventional extrusion process as described above. PTFE exits the extruder tube in a tubular structure having an approximate thickness of 170 µ (± 15 µ) before stretching. It is presumed that the thickness throughout the tubular structure is fairly uniform, yet not uniform enough to provide consistent radial strength through the structure without crimping thereof.

After the green tube is extruded, it is then expanded to form ePTFE. The tube is extended over a mandrel of approximately equal diameter to the extruded tube and clamped at both extremities. The tube is heat soaked at 533K (500°F) to soften the material and stretched at 533K (500°F) at the rate of 35 cm/sec. The tube is then partially sintered at 622K (660°F) for 14 minutes to 900% length or 1500% length. The tube is cooled at 291K (65°F) for about 5 minutes, then cooled at room temperatures for about 10 minutes. The tube is removed from the mandrel, inspected and inventoried for further manufacturing and use.

Measurement of the thickness of the tube can be accomplished by a plurality of methods. Two of the predominant methods of measuring thickness are the Thwing method and the snap-gauge technique, both of which are well-known in the art. After expansion, the thickness of the outer tube (1500% length) using the Thwing method is approximately .040mm while that of the inner tube (900% length) is approximately .070 mm. Using the snap-gauge technique, the thickness of the outer tube is .128 mm and that of the inner tube is .138 mm. Knowing the different results produced by the two methods is important in determining the applicability of the manufactured stent-graft prosthesis.

The stent is affixed to the exterior surface of a balloon on a balloon catheter so as to frictionally fit thereover. The balloon is inflated somewhat so as to expand the stent slightly. The previously fabricated outer tube is then placed into a catheter with one extremity of the tube extended past and folded over the periphery of the catheter. The balloon with the stent is then inserted into the catheter having the tube therein, after which the balloon is deflated so that the stent retains its expanded configuration. While the two components are in the catheter, an inner tube is radially compacted and inserted into the stent having the outer tube thereon. The inner tube is unfolded inside of the catheter (i.e, via a set of tweezers or similar device) and a mandrel is placed inside the tube-stent-tube combination, forcing the inner tube into a cylindrical configuration.

A heat shrink tube such as a silicone tube is subsequently placed over the entire structure, and ePTFE tape is used to tightly wrap the extremities of the heat shrink tube. The tube is then heat shrunk in a heated oven at elevated pressures, promoting conformance of the tube with the outer surface of the structure and initiating the sintering process wherein lamination occurs. The heat shrink tube is removed from the oven and permitted to cool. The ends of the PTFE tube(s) laminate are thereafter cut to match the contour of the stent, a process known as "scalloping" or "routing".

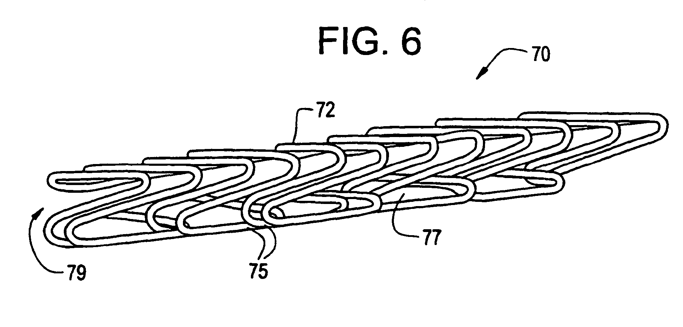

Although a wide variety of stents may be used, Figure 6 shows a perspective view of one particular stent which may be employed between outer tube 12 and inner tube 14 of prosthesis 10. The particular stent shown in Figure 6 is more fully described in commonly assigned U.S. Patent No. 5,575,816 to Rudnick, et al. Stent 70 is an intraluminally implantable stent formed of helically wound wire. Multiple windings 75 of a single metallic wire 72, preferably composed of a temperature-sensitive material such as Nitinol, provide stent 70 with a generally elongate tubular configuration which is radially expandable after implantation in a body vessel. The multiple windings 75 of stent 70 define open spaces 77 throughout the tubular configuration and define a central open passage 79 therethrough. The helically wound wire configuration not only ensures patency and flexibility, but the open spaces also allow adhering of the two tubular layers therethrough. Although this particular stent construction is shown and described with reference to the present invention, any stent of similar construction configured for the use anticipated herein may be utilized.

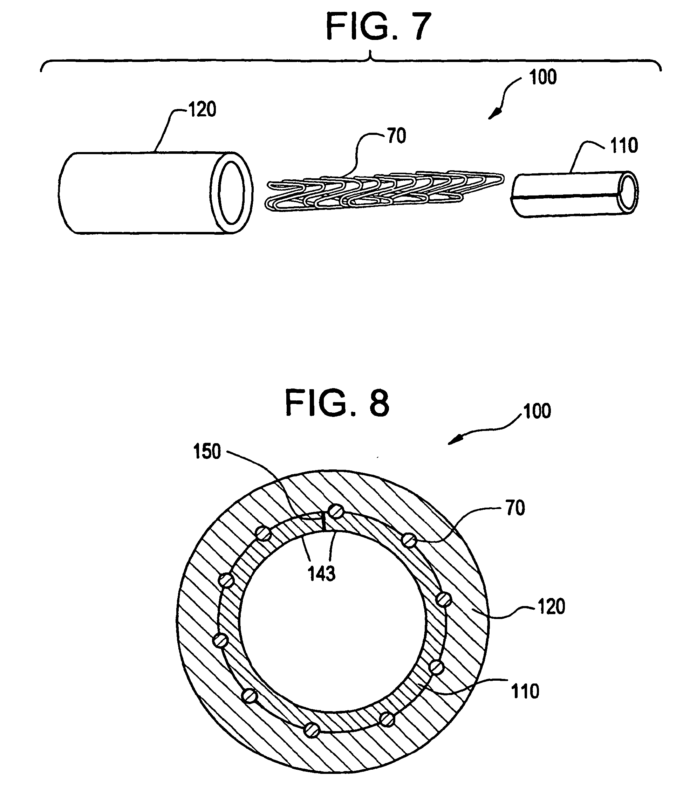

One embodiment of the present invention will now be explained with reference to figures 7, 8. A graft-stent composite device 100 has an inner layer 110 comprising a tubular-configured ePTFE sheet such as shown and described with respect to Figure 4 above. Inner layer 110 has a thickness t sufficient for insertion into a stent 70. Stent 70 is correspondingly sized and shaped for insertion into tube 120 formed of an extruded and expanded PTFE such as that shown and described with respect to Figure 2 above. Referring to Figure 8, one benefit of having sheet 110 on the interior of the stent 70 is to eliminate the need to close sheet 110, thereby obviating any overlap or seams which may increase the thickness of the graft. Accordingly, sheet 110 is depicted as having longitudinal edges 143 with a gap region 150 defined therebetween, indicating the lack of adherence between the edges. In the alternative according to the invention, edges 143 could be secured to one another before insertion of the tubular structure into stent 70 via application of a thermoplastic adhesive to the contacting surfaces of edges 143.

The stent may be adhered to the inner PTFE tubular layer, the outer PTFE tubular layer, or both. Such adherence may be effected with or without the use of an adhesive. Additionally, when a stent with a plurality of open spaces or slots therethrough (such as wire stent 70) is utilized in the prosthesis 10 or device 100, the inner and outer tubular layers may be adhered to each other through the spaces in the stent. Such adherence may be accomplished with the use of an adhesive. Alternatively, the tubular layers may be adhered directly together through the spaces by the lamination of the layers. Sintering is one method of effecting such adherence.

Various changes and modifications can be made to the present invention. It is intended that all such changes and modifications come within the scope of the invention as set forth in the following claims.

## Claims

1. A stent-graft composite intraluminal prosthetic device (10;100) comprising:
an elongated radially adjustable tubular stent (17;70), defining opposed interior and exterior stent surfaces;
a PTFE stent cover (12;120) positioned about the exterior surface of said stent; and
a PTFE stent liner (14;110) positioned about the interior surface of said stent;
wherein one of said cover and said liner is formed of a seamless extruded tube and the other of said cover and said liner is formed of a sheet,
wherein the sheet (40) has longitudinal edges (43;143),
**characterized in that** said liner (110) is formed from said sheet and has said longitudinal edges (43;143) joined so as to not overlap, contacting surfaces of said longitudinal edges being adhered to one another.

2. The stent-graft composite device of claim 1, wherein said cover (12;120) and said liner (14;110) are formed of ePTFE having a porous structure defined by nodes and fibrils.

3. The stent-graft composite device of claim 1, wherein said stent (17;70) comprises a tubular configuration having a plurality of open spaces between said opposed interior and exterior stent surfaces, said liner and said cover being secured together through said open spaces of said stent.

4. The stent-graft composite device of claim 3, wherein said liner and said cover are adheringly secured through said open spaces of said stent.

5. The stent-graft composite device of claim 3, wherein said liner and said cover are laminated together through said open spaces of said stent.

6. The stent-graft composite device of claim 1, wherein said liner is adheringly secured to said inner surface of said stent, said cover being adheringly secured to said outer surface of said stent.

7. The stent-graft composite device of claim 1, wherein said stent (17;70) comprises a helically wound wire.

8. A method of forming a stent-graft prosthesis (10;100) comprising the steps of:
providing a first PTFE tubular structure (14;110) comprising an interior surface and an exterior surface;
placing a tubular diametrically deformable stent (17;70) circumferentially around the exterior surface of the first tubular structure; and
disposing a second PTFE tubular structure (12;120) externally about the stent,
wherein one of the first and second PTFE tubular structures is a seamless extruded PTFE tube and the other of the first and second PTFE tubular structures is formed from a sheet,
wherein said sheet (49) has longitudinal edges (43;143),
**characterized in that** the first PTFE tubular structure (14;110) is formed from the sheet such that said longitudinal edges (43;143) are joined so as to not overlap, contacting surfaces of said longitudinal edges being adhered to one another.

9. The method of claim 8, wherein said providing step comprises providing the first PTFE tubular structure (14;110) formed of ePTFE having a porous structure defined by nodes and fibrils,
said disposing step comprising providing the second PTFE tubular structure (12;120) formed of ePTFE having a porous structure defined by nodes and fibrils.

10. The method of claim 8, wherein said placing step comprises placing a tubular diametrically deformable stent (17;70) having a plurality of open spaces between the interior and exterior surfaces of the stent circumferentially around the exterior surface of the first tubular structure (14;110),
said method further comprising securing the first PTFE tubular structure to the second PTFE tubular structure (12;120) through the open spaces of the stent.

11. The method of claim 10, wherein the first PTFE structure is adheringly secured to the second PTFE tubular structure through the open spaces of the stent.

12. The method of claim 10, wherein the first PTFE tubular structure and the second PTFE tubular structure are laminated together through the open spaces of the stent.

13. The method of claim 8, wherein the stent (17;70) has opposed interior and exterior surfaces,
said method further comprising adheringly securing the first PTFE tubular structure to the interior surface of the stent,
said method further comprising adheringly securing the second PTFE tubular structure to the exterior surface of the stent.

14. The method of claim 8, wherein said placing step comprises placing a tubular diametrically deformable stent (17;70) comprising a helically wound wire circumferentially around the exterior surface of the first tubular structure.

## Patentansprüche

1. Intraluminale prothetische Stent-Graft-Kompositvorrichtung (10; 100), umfassend:
einen länglichen radial anpassbaren röhrenförmigen Stent (17; 70), der eine innere und eine gegenüberliegende äußere Stent-Fläche bestimmt;
eine PTFE-Stent-Abdeckung (12; 120), die um die äußere Fläche des Stents positioniert ist; und
ein PTFE-Stent-Einsatzstück (14; 110), das um die innere Fläche des Stents positioniert ist;
wobei entweder die Abdeckung oder das Einsatzstück aus einer nahtlos stranggepressten Röhre gebildet ist und die/das andere, die Abdeckung oder das Einsatzstück, aus einem Blech gebildet ist,
wobei das Blech (40) Längskanten (43; 143) aufweist,
**dadurch gekennzeichnet, dass** das Einsatzstück (110) aus dem Blech gebildet ist und seine Längskanten (43; 143) so verbunden sind, dass sie nicht überlappen, wobei die sich berührenden Flächen der Längskanten miteinander verklebt sind.

2. Stent-Graft-Kompositvorrichtung nach Anspruch 1, wobei die Abdeckung (12; 120) und das Einsatzstück (14; 110) aus ePTFE gebildet sind, das eine poröse Struktur aufweist, die durch Knoten und Fibrillen bestimmt ist.

3. Stent-Graft-Kompositvorrichtung nach Anspruch 1, wobei der Stent (17; 70) eine röhrenförmige Ausgestaltung umfasst, die eine Mehrzahl offener Räume zwischen der inneren und der gegenüberliegenden äußeren Stent-Fläche aufweist, wobei das Einsatzstück und die Abdeckung durch die offenen Räume des Stents aneinander gesichert sind.

4. Stent-Graft-Kompositvorrichtung nach Anspruch 3, wobei das Einsatzstück und die Abdeckung durch die offenen Räume des Stents klebend gesichert sind.

5. Stent-Graft-Kompositvorrichtung nach Anspruch 3, wobei das Einsatzstück und die Abdeckung durch die offenen Räume des Stents miteinander laminiert sind.

6. Stent-Graft-Kompositvorrichtung nach Anspruch 1, wobei das Einsatzstück klebend an der inneren Fläche des Stents gesichert ist, wobei die Abdeckung klebend an der äußeren Fläche des Stents gesichert ist.

7. Stent-Graft-Kompositvorrichtung nach Anspruch 1, wobei der Stent (17; 70) einen schraubenförmig gewundenen Draht umfasst.

8. Verfahren zum Bilden einer Stent-Graft-Prothese (10; 100), umfassend die folgenden Schritte:
Bereitstellen einer ersten röhrenförmigen PTFE-Struktur (14; 110), umfassend eine innere Fläche und eine äußere Fläche;
Platzieren eines röhrenförmigen diametral verformbaren Stents (17; 70) in Umfangsrichtung um die äußere Fläche der ersten röhrenförmigen Struktur; und
Anordnen einer zweiten röhrenförmigen PTFE-Struktur (12; 120) außen um den Stent,
wobei eine, die erste oder die zweite röhrenförmige PTFE-Struktur, eine nahtlos stranggepresste PTFE-Röhre ist und die andere, die erste oder die zweite röhrenförmige PTFE-Struktur, aus einem Blech gebildet ist,
wobei das Blech (49) Längskanten (43; 143) aufweist,
**dadurch gekennzeichnet, dass** die erste röhrenförmige PTFE-Struktur (14; 110) so aus dem Blech gebildet ist, dass die Längskanten (43; 143) so verbunden sind, dass sie nicht überlappen, wobei die sich berührenden Flächen der Längskanten miteinander verklebt sind.

9. Verfahren nach Anspruch 8, wobei der Schritt des Bereitstellens das Bereitstellen der ersten röhrenförmigen PTFE-Struktur (14; 110) umfasst, die aus ePTFE gebildet ist, das eine poröse Struktur aufweist, die durch Knoten und Fibrillen bestimmt ist,
wobei der Schritt des Anordnens das Bereitstellen der zweiten röhrenförmigen PTFE-Struktur (12; 120) umfasst, die aus ePTFE gebildet ist, das eine poröse Struktur aufweist, die durch Knoten und Fibrillen bestimmt ist.

10. Verfahren nach Anspruch 8, wobei der Schritt des Platzierens das Platzieren eines röhrenförmigen diametral verformbaren Stents (17; 70) umfasst, der eine Mehrzahl offener Räume zwischen der inneren und der äußeren Fläche des Stents in Umfangsrichtung um die äußere Fläche der ersten röhrenförmigen Struktur (14; 110) aufweist,
wobei das Verfahren überdies das Sichern der ersten röhrenförmigen PTFE-Struktur an der zweiten röhrenförmigen PTFE-Struktur (12; 120) durch die offenen Räume des Stents umfasst.

11. Verfahren nach Anspruch 10, wobei die erste PTFE-Struktur durch die offenen Räume des Stents klebend an der zweiten röhrenförmigen PTFE-Struktur gesichert ist.

12. Verfahren nach Anspruch 10, wobei die erste röhrenförmige PTFE-Struktur und die zweite röhrenförmigen PTFE-Struktur durch die offenen Räume des Stents miteinander laminiert sind.

13. Verfahren nach Anspruch 8, wobei der Stent (17; 70) eine innere und eine gegenüberliegende äußere Flächen aufweist,
wobei das Verfahren überdies das klebende Sichern der ersten röhrenförmigen PTFE-Struktur an der inneren Fläche des Stents umfasst,
wobei das Verfahren überdies das klebende Sichern der zweiten röhrenförmigen PTFE-Struktur an der äußeren Fläche des Stents umfasst.

14. Verfahren nach Anspruch 8, wobei der Schritt des Platzierens das Platzieren eines röhrenförmigen diametral verformbaren Stents (17; 70) umfasst, der einen schraubenförmig gewundenen Draht in Umfangsrichtung um die äußere Fläche der ersten röhrenförmigen Struktur umfasst.

## Revendications

1. Dispositif composite de greffe d'endoprothèse intraluminal prosthétique (10 ; 100) comprenant:
une endoprothèse tubulaire (17 ; 70) allongée réglable radialement, définissant des surfaces d'endoprothèse intérieure et extérieure opposées ;
une enveloppe d'endoprothèse en PTFE (12 ; 120) positionnée autour de la surface extérieure de ladite endoprothèse ; et
un revêtement d'endoprothèse en PTFE- (14 ; 110) positionné autour de la surface intérieure de ladite endoprothèse ;
dans lequel l'un parmi ladite enveloppe et ledit revêtement est formé d'un tube extrudé sans soudure et l'autre parmi ladite enveloppe et ledit revêtement est formé d'une feuille,
dans lequel la feuille (40) présente des bords longitudinaux (43 ; 143),
**caractérisé en ce que** ledit revêtement (110) est formé à partir de ladite feuille et présente lesdits bords longitudinaux (43 ; 143) joints de manière à ne pas se chevaucher, les surfaces en contact desdits bords longitudinaux étant collées l'une à l'autre.

2. Dispositif composite de greffe d'endoprothèse selon la revendication 1, dans lequel ladite enveloppe (12 ; 120) et ledit revêtement (14 ; 110) sont formés d'ePTFE présentant une structure poreuse définie par des noeuds et fibrilles.

3. Dispositif composite de greffe d'endoprothèse selon la revendication 1, dans lequel ladite endoprothèse (17 ; 70) comprend une configuration tubulaire présentant une multiplicité d'espaces ouverts entre lesdites surfaces d'endoprothèse intérieure et extérieure opposées, ledit revêtement et ladite enveloppe étant fixés ensemble à travers lesdits espaces ouverts de ladite endoprothèse.

4. Dispositif composite de greffe d'endoprothèse selon la revendication 3, dans lequel ledit revêtement et ladite enveloppe sont fixés de manière adhérente à travers lesdits espaces ouverts de ladite endoprothèse.

5. Dispositif composite de greffe d'endoprothèse selon la revendication 3, dans lequel ledit revêtement et ladite enveloppe sont stratifiés ensemble à travers lesdits espaces ouverts de ladite endoprothèse.

6. Dispositif composite de greffe d'endoprothèse selon la revendication 1, dans lequel ledit revêtement est fixé de manière adhérente à ladite surface intérieure de ladite endoprothèse, ladite enveloppe étant fixée de manière adhérente à ladite surface extérieure de ladite endoprothèse.

7. Dispositif composite de greffe d'endoprothèse selon la revendication 1, dans lequel ladite endoprothèse (17 ; 70) comprend un fil enroulé de manière hélicoïdale.

8. Procédé pour former une prothèse de greffe d'endoprothèse (10 ; 100) comprenant les étapes consistant à :
mettre à disposition une première structure tubulaire en PTFE (14; 110) comprenant une surface intérieure et une surface extérieure ;
placer une endoprothèse tubulaire au diamètre déformable (17 ; 70) de manière circonférentielle autour de la surface extérieure de la première structure tubulaire ; et
disposer une seconde structure tubulaire en PTFE (12 ; 120) extérieurement autour de l'endoprothèse,
dans laquelle l'une parmi les première et seconde structures tubulaires en PTFE est un tube en PTFE extrudé sans soudure et l'autre parmi les première et seconde structures tubulaires en PTFE est formée à partir d'une feuille,
dans laquelle ladite feuille (49) présente des bords longitudinaux (43 ; 143),
**caractérisée en ce que** la première structure tubulaire en PTFE- (14 ; 110) est formée à partir de la feuille de sorte que lesdits bords longitudinaux (43 ; 143) sont joints de manière à ne pas se chevaucher, les surfaces en contact desdits bords longitudinaux étant collées l'une à l'autre.

9. Méthode selon la revendication 8, dans laquelle ladite étape de mise à disposition consiste à mettre à disposition la première structure tubulaire en PTFE (14 ; 110) formée à partir d'ePTFBE présentant une structure poreuse définie par des noeuds et fibrilles,
ladite étape consistant à disposer comprenant la mise à disposition de la seconde structure tubulaire en PTFE- (12 ; 120) formée d'ePTFE présentant une structure poreuse définie par des noeuds et fibrilles.

10. Méthode selon la revendication 8, dans laquelle ladite étape consistant à placer comprend le placement d'une endoprothèse tubulaire au diamètre déformable (17 ; 70) présentant une multiplicité d'espaces ouverts entre les surfaces intérieure et extérieure de l'endoprothèse de manière circonférentielle autour de la surface extérieure de la première structure tubulaire (14 ; 110),
ladite méthode comprenant en outre la fixation de la première structure tubulaire en PTFE à la seconde structure tubulaire en PTFE (12 ; 120) à travers les espaces ouverts de l'endoprothèse.

11. Méthode selon la revendication 10, dans laquelle la première structure en PTFE est fixée de manière adhérente à la seconde structure tubulaire en PTFE à travers les espaces ouverts de l'endoprothèse.

12. Méthode selon la revendication 10, dans laquelle la première structure tubulaire en PTFE et la seconde structure tubulaire en PTFE- sont stratifiées ensemble à travers les espaces ouverts de l'endoprothèse.

13. Méthode selon la revendication 8, dans laquelle l'endoprothèse (17 ; 70) présente des surfaces intérieure et extérieure opposées,
ladite méthode comprenant en outre la fixation de manière adhérente de la première structure tubulaire en PTFE à la surface intérieure de l'endoprothèse,
ladite méthode comprenant en outre la fixation de manière adhérente de la seconde structure tubulaire en PTFE à la surface extérieure de l'endoprothèse.

14. Méthode selon la revendication 8, dans laquelle ladite étape consistant à placer comprend le placement d'une endoprothèse tubulaire au diamètre déformable (17 ; 70) comprenant un fil enroulé de manière hélicoïdale de manière circonférentielle autour de la surface extérieure de la première structure tubulaire.
